(19) Europäisches Patentamt
European Patent Office
Office européen des brevets

(11) Veröffentlichungsnummer: **0 026 899**
**A1**

# (12) EUROPÄISCHE PATENTANMELDUNG

(21) Anmeldenummer: **80105887.6**

(22) Anmeldetag: **29.09.80**

(51) Int. Cl.³: **A 61 K 31/48**, C 07 D 519/02, C 07 D 498/14 // (C07D498/14, 263/00, 241/00, 209/00)

(30) Priorität: **09.10.79 CH 9096/79**

(43) Veröffentlichungstag der Anmeldung: **15.04.81 Patentblatt 81/15**

(84) Benannte Vertragsstaaten: **AT BE CH DE FR GB IT LI LU NL SE**

(71) Anmelder: **SANDOZ AG, Lichtstrasse 35, CH-4002 Basel (CH)**

(72) Erfinder: **Pfäffli, Paul, Dr., Auf den Hallen 18, CH-4104 Oberwil (CH)**
Erfinder: **Hauth, Hartmut, Dr., Burgstrasse 2, CH-4125 Riehen (CH)**
Erfinder: **Stütz, Peter, Dr., Nästlbergergasse 15, A-1130 Wien (AT)**

(54) **Peptidergotalkaloide, Verfahren zu deren Herstellung, diese enthaltende pharmakologische Zusammensetzungen und ihre Anwendung bei der therapeutischen Behandlung.**

(57) Die Erfindung betrifft auf der Amidbrücke zwischen dem Peptidteil und dem Lysergsäurederivatrest durch einen linearen aliphatischen Kohlenwasserstoffrest substituierte Peptidergotalkaloide sowie ihre Herstellung durch Kondensation eines Säureadditionssalzes eines entsprechend N-substituierten Aminocyclols mit einem entsprechenden reaktiven Lysergsäurederivat oder Reduktion eines entsprechenden, in 9,10-Stellung ungesättigten Peptidergotalkaloids, wobei die in 9,10-Stellung gesättigten Verbindungen erhalten werden. Die Verbindungen der Erfindung können als Arzneimittel für die Indikation Zerebralinsuffizienz verwendet werden.

EP 0 026 899 A1

SANDOZ AG, 4002 Basel Case 100-5266

Peptidergotalkaloide, Verfahren zu deren Herstellung, diese enthaltende pharmakologische Zusammensetzungen und ihre Anwendung bei der therapeutischen Behandlung

Gegenstand der vorliegenden Erfindung sind Peptidergotalkaloide, Verfahren zu deren Herstellung, pharmazeutische Zusammensetzungen, die diese Peptidergotalkaloide enthalten, sowie die Anwendung dieser Peptidergotalkaloide bei der therapeutischen Behandlung.

Die Erfindung betrifft insbesondere auf der Amidbrücke zwischen dem Peptidteil und dem Lysergsäurederivatrest durch einen linearen aliphatischen Kohlenwasserstoffrest substituierte Peptidergotalkaloide und ihre Säureadditionssalze.

Diese Verbindungen, im folgenden als erfindungsgemässe Verbindungen bezeichnet, umfassen die in Stellung 19 wie oben angegeben substituierten Derivate der sowohl in der Natur vorkommenden wie auch synthetisch zugänglichen Peptidergotalkaloide. Sie können ferner in irgendwelcher Stellung substituiert sein. Der Kohlenwasserstoffrest auf der Amidbrücke kann gesättigt oder ungesättigt sein.
Insbesondere werden Verbindungen der Formel I umfasst,

BAD ORIGINAL

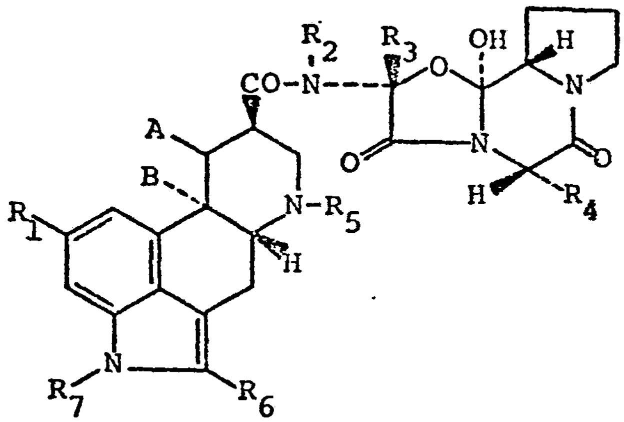

I

worin

$R_1$ für Wasserstoff oder Brom steht,

$R_2$ und $R_3$ unabhängig voneinander eine lineare Alkylgruppe mit 1 bis 4 Kohlenstoffatomen bedeuten,

$R_4$ für Alkyl mit 1 bis 6 Kohlenstoffatomen oder Benzyl steht,

$R_5$ für Alkyl mit 1 bis 4 Kohlenstoffatomen steht,

$R_6$ Wasserstoff oder Halogen mit einer Atomzahl von 9 bis 35 bedeutet,

$R_7$ für Wasserstoff oder Alkyl mit 1 bis 4 Kohlenstoffatomen steht und

A und B zusammen eine einfache Bindung bilden oder

A und B je für Wasserstoff stehen oder

A Wasserstoff und B Methoxy darstellen,

und ihre Säureadditionssalze.

In der Formel I steht $R_1$ vorzugsweise für Wasserstoff. $R_2$ bedeutet vorzugsweise die Methylgruppe. $R_3$ bedeutet beispielsweise Methyl oder Aethyl, vorzugsweise Aethyl. $R_4$ steht beispielsweise für Benzyl, vorzugsweise für verzweigtes Alkyl mit 3 oder 4 Kohlenstoffatomen. $R_5$ bedeutet beispielsweise eine n- oder Isopropyl-, vorzugsweise eine Methylgruppe. $R_6$ steht zweckmässigerweise für Wasserstoff; falls der Substituent $R_6$ Halogen bedeutet, stellt er bevorzugt Brom dar. Vorzugsweise

0026899

bedeutet $R_7$ Wasserstoff. A und B stehen zweckmässigerweise je für Wasserstoff.

Gemäss der vorliegenden Erfindung gelangt man zu den erfindungsgemässen Verbindungen, indem man

a) ein Säureadditionssalz eines entsprechend N-substituierten Aminocyclols mit einem entsprechenden reaktiven Lysergsäurederivat kondensiert oder

b) zur Herstellung der in 9,10-Stellung gesättigten Verbindungen, die frei von unter den Reduktionsbedingungen empfindlichen Substituenten sind, die entsprechenden, in 9,10-Stellung ungesättigten Verbindungen reduziert

und die erhaltenen Verbindungen gewünschtenfalls in ihre Säureadditionssalze überführt.

Insbesondere gelangt man zu den Verbindungen der Formel I und ihren Säureadditionssalzen, indem man

a) ein Säureadditionssalz einer Verbindung der Formel II,

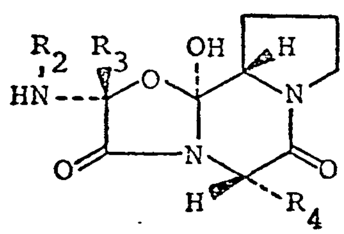

II

worin $R_2$, $R_3$ und $R_4$ obige Bedeutung besitzen, mit einem reaktiven Säurederivat einer Verbindung der Formel III,

 

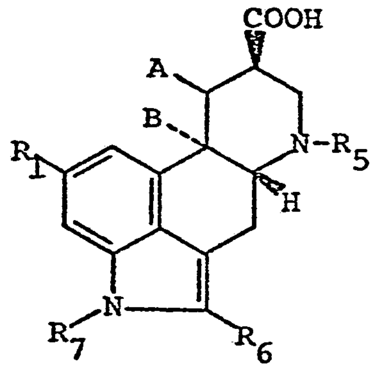

III

worin $R_1$, $R_5$, $R_6$, $R_7$, A und B obige Bedeutung besitzen, kondensiert oder

b) zur Herstellung von Verbindungen der Formel Ia,

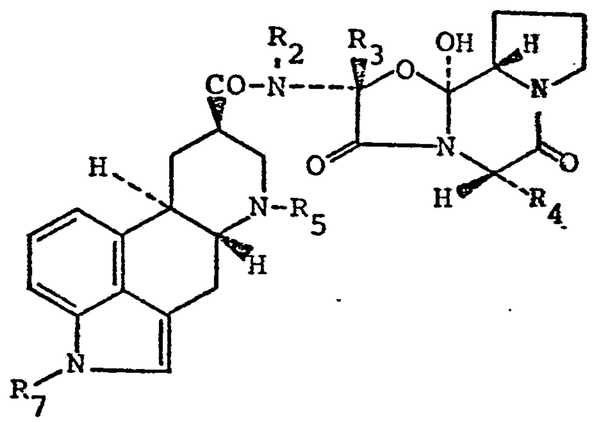

Ia

worin $R_2$, $R_3$, $R_4$, $R_5$ und $R_7$ obige Bedeutung besitzen, Verbindungen der Formel Ib,

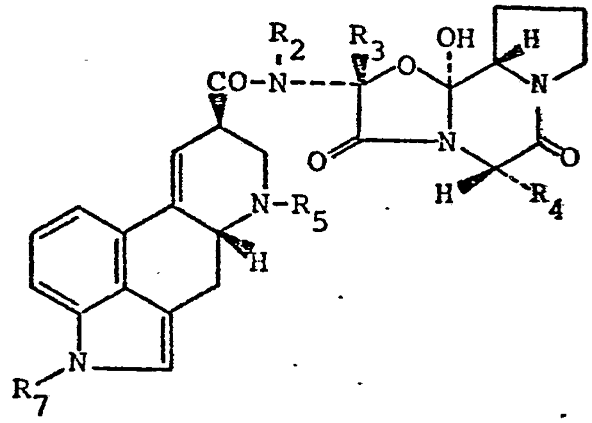

Ib

worin $R_2$, $R_3$, $R_4$, $R_5$ und $R_7$ obige Bedeutung besitzen, reduziert

und die erhaltenen Verbindungen gewünschtenfalls in ihre Säureadditionssalze überführt.

Das erfindungsgemässe Verfahren a) kann analog zu bekannten Methoden für die Kondensation zur Herstellung von analogen Peptidergotalkaloiden erfolgen.

Ein geeignetes Säureadditionssalz des Aminocyclols ist das Hydrochlorid oder das Methansulfonat.

Als reaktive Derivate des Lysergsäurederivates können beispielsweise das Säurechlorid, das Säureazid oder das gemischte Anhydrid mit Schwefelsäure oder Trifluoressigsäure eingesetzt werden.

Man kann auch das Additionsprodukt des Lysergsäurederivates mit Dimethylformamid und Thionylchlorid, Phosgen oder Oxalylchlorid verwenden. Vorzugsweise wird die Reaktion in Gegenwart von Triäthylamin oder Pyridin durchgeführt. Geeignete Lösungsmittel sind z.B. Chloroform, Methylenchlorid, Dimethylformamid oder Acetonitril.

Die Umsetzung kann bei Temperaturen zwischen - 30 ° bis + 20 ° C durchgeführt werden.

Die Reduktion gemäss Verfahren b) kann auf an sich bekannte Weise erfolgen, beispielsweise durch katalytische Hydrierung, z.B. unter Verwendung von Palladium auf Aluminiumoxid oder Kohle, oder von Nickel oder Platin als Hydrierungskatalysator. Die katalytische Hydrierung

kann in einem unter den Reaktionsbedingungen inerten Lösungsmittel durchgeführt werden, vorzugsweise bei Raumtemperatur und Normaldruck.

Die erfindungsgemässen Verbindungen können auf an sich bekannte Weise aus dem Reaktionsgemisch isoliert und gereinigt werden.

Die freien Basen können in Säureadditionssalze umgewandelt werden und umgekehrt. Für die Salzbildung geeignete Säuren sind z.B. die Chlorwasserstoffsäure, Schwefelsäure, Maleinsäure, Fumarsäure und Weinsäure.

Die Ausgangsverbindungen der Formel II können ausgehend von der Verbindung der Formel IV,

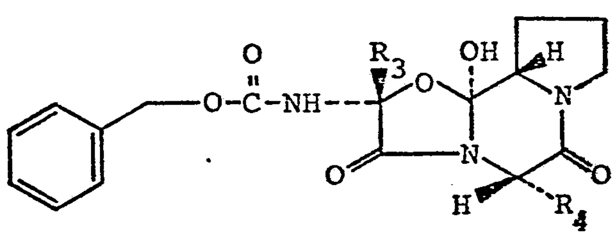

IV

worin $R_3$ und $R_4$ obige Bedeutung besitzen, nach an sich bekannten Methoden hergestellt werden, beispielsweise durch N-Alkylierung, z.B. mittels Alkylhalogeniden oder Fluorsulfonsäureester, und anschliessende Hydrierung.

Soweit die Herstellung der Ausgangsprodukte nicht beschrieben wird, sind diese bekannt oder nach an sich bekannten Verfahren bzw. analog zu an sich bekannten Verfahren herstellbar.

Die erfindungsgemässen Verbindungen weisen im Tierversuch interessante pharmakodynamische Eigenschaften auf. Sie können daher als Heilmittel verwendet werden.

In vitro hemmen sie die noradrenalinabhängige cAMP (cyclic adenosine 3',5'-monophosphate)-Bildung im Rattenhirn im Konzentrationsbereich 0,01 - 0,1 $\mu$ M [Lit.: R. Markstein and H. Wagner, Gerontology 24 (Suppl. 1), 94 - 105 (1978)]. An der Dopamin-sensitiven Adenylatcyclase in Homogenat aus Rattenstriaten [R. Markstein and coll., Journal of Neurochemistry 31, 1163 - 1172 (1978)] zeigen sie im Konzentrationsbereich 0,1 - 10 $\mu$ M stimulierende Wirkungen. An der Serotonin-sensitiven Adenylatcyclase [J.K. Northup and coll., Molecular Pharmacology 14, 804 - 819 (1978)], wirken sie serotoninagonistisch. Letztere Wirkung wurde am Schlaf/Wachzyklus der chronisch implantierten Ratte im Dosenbereich von 3 - 30 mg/kg p.o. bzw. 1 - 10 mg/kg i.p. bestätigt, indem die Verbindungen den Wachanteil steigern und den Paradoxalschlaf reduzieren. Damit erweisen sich die erfindungsgemässen Verbindungen als zentrale $\alpha$-Blocker mit dopamino- und serotoninomimetischen Wirkungen.
In vivo hemmen sie mit einer Dosis von 2 mg/kg i.p. den initialen cGMP (cyclic guanosine 3',5'-monophosphate)-Anstieg im Cerebellum von Ratten unter Stresseinfluss [Lit.: V. Dinnendahl and K. Stock, Naunyn-Schmiedeberg's Arch. Pharmacol. 290, 297 - 306 (1975)]. Dieser Befund zeigt, dass sie eine gute Gehirngängigkeit aufweisen.

Aufgrund dieser Eigenschaften können die neuen Substanzen für die Indikation Zerebralinsuffizienz verwendet werden.

Sie können allein oder in geeigneter Dosierungsform verabreicht werden. Die Arzneiformen, beispielsweise eine Tablette, können analog zu bekannten Methoden hergestellt werden.

Für die oben genannte Anwendung ist eine täglich zu verabreichende Menge zwischen 2 und 500 mg Wirkstoff angezeigt. Diese Dosis kann auch in kleineren Dosen 2 - 4 mal täglich oder in Retardform verabreicht werden. Eine Einheitsdosis kann zwischen 0,5 und 250 mg des Wirkstoffes zusammen mit geeigneten pharmazeutisch indifferenten Hilfsstoffen enthalten.

Gegenstand der Erfindung sind auch pharmazeutische Zusammensetzungen, die die erfindungsgemässen Verbindungen enthalten. Für ihre Herstellung können die in der Pharmazie gebräuchlichen Hilfs- und Trägerstoffe verwendet werden.

Ferner betrifft die Erfindung die Anwendung der neuen Substanzen als Pharmazeutika, beispielsweise bei der therapeutischen Behandlung, insbesondere zur Behandlung der Zerebralinsuffizienz.

In den nachfolgenden Beispielen, die die Erfindung näher erläutern, erfolgen alle Temperaturangaben in Celsiusgraden und sind unkorrigiert.

Das Beispiel 1 betrifft die bevorzugte Verbindung.

Beispiel 1: 2'β-Aethyl-9,10α-dihydro-5'α-isopropyl-19-methyl-ergopeptin (Verfahren a)

100 ml abs. Dimethylformamid werden unter einer Stickstoffatmosphäre auf - 20 ° gekühlt, dazu innert 10 Minuten unter Rühren bei - 20 ° und entsprechender Kühlung eine Lösung von 2,58 ml (30 m Mol) Oxalylchlorid in 10 ml abs. Acetonitril getropft und die entstandene Suspension 10 Minuten bei - 20 ° weitergerührt. Nach Zugabe von 8,11 g (30 m Mol) fester, bei 120 ° im Hochvakuum getrockneter 9,10α-Dihydrolysergsäure und 30-minütigem Rühren bei 0 ° wird auf - 10 ° abgekühlt, innert 5 Minuten 25 ml (310 m Mol) abs. Pyridin bei - 10 ° und entsprechender Kühlung zugetropft und 4,07 g (10 m Mol) festes (2R,5S,10aS,10bS)-2-Aethyl-tetrahydro-10b-hydroxy-5-isopropyl-2-methylamino-8H-oxazolo[3,2-a]pyrrolo[2,1-c]pyrazin-3,6(2H,5H)-dion monomethansulfonat zugegeben. Das Reaktionsgemisch wird innert 2,5 Stunden von - 10 ° auf 0 ° erwärmen gelassen, wieder auf - 20 ° gekühlt, innert 10 Minuten unter Rühren bei - 20 ° mit 30 ml ca. 0,05-molarem Citratpuffer von pH 4, dann 50 ml Methylenchlorid vermischt und zwischen Methylenchlorid/Aethanol (90 : 10) und 2N Natriumcarbonat verteilt. Nach Trocknung der organischen Phasen mit Natriumsulfat und Eindampfen hinterbleibt ein amorpher Schaum. Die so erhaltene Titelverbindung wird aus Methanol kristallisiert und umkristallisiert. Smp. ca. 180 ° (Zers.); $[\alpha]_D^{20}$ = + 53,9 ° (0,5 % in Chloroform).

Das Ausgangsmaterial kann wie folgt hergestellt werden:

a) (2R,5S,10aS,10bS)-2-Aethyl-octahydro-10b-hydroxy-5-isopropyl-3,6-dioxo-8H-oxazolo[3,2-a]pyrrolo[2,1-c]pyrazin-2-(N-methyl-carbaminsäure)benzylester

Zu einer Lösung von 2,35 ml (25 m Mol) Tertiärbutylalkohol in 40 ml abs. Tetrahydrofuran werden unter Stickstoffatmosphäre und Eiskühlung 14,3 ml einer 1,75-molaren (25 m Mol) Lösung von Butyllithium in Hexan getropft, 4,31 g festes (2R,5S,10aS,10bS)-2-Aethyl-octahydro-10b-hydroxy-5-isopropyl-3,6-dioxo-8H-oxazolo[3,2-a]pyrrolo[2,1-c]pyrazin-2-carbaminsäure benzylester (10 m Mol) bei 0 ° zugegeben, auf - 20 ° gekühlt, innert 15 Minuten bei - 20 ° 0,83 ml (10 m Mol) Fluorsulfonsäure-methylester zugetropft und 30 Minuten bei - 20 ° ausreagieren gelassen. Anschliessend wird noch dreimal folgende Prozedur bei - 20 ° wiederholt: Zutropfen von 0,47 ml (5 m Mol) Tertiärbutylalkohol, 2,86 ml einer 1,75-molaren (5 m Mol) Lösung von Butyllithium in Hexan, innert 5 Minuten 0,42 ml (5 m Mol) Fluorsulfonsäure-methylester und 30 Minuten Ausreagieren unter Rühren. Bei - 20 bis - 10 ° werden anschliessend 20 ml 2 M Ammoniumsulfat-Lösung zugetropft, das Gemisch zwischen Toluol und Wasser verteilt, die organischen Phasen mit Natriumsulfat getrocknet, eingedampft und im Hochvakuum bei 22 ° getrocknet. Der Rückstand wird aus Toluol/Cyclohexan (20:80) kristallisiert, wobei 1-Aequivalent Cyclohexan-enthaltende Kristalle von (2R,5S,10aS,10bS)-2-Aethyl-octahydro-10b-hydroxy-5-isopropyl-3,6-dioxo-8H-oxazolo[3,2-a]pyrrolo[2,1-c]pyrazin-2-(N-methyl-carbaminsäure)benzylester resultieren. Smp.: 60 -

BAD ORIGINAL

75 ° (unter Lösungsmittel-Verlust); $[\alpha]_D^{20}$ = + 36,1 ° (1,07 % in Chloroform).

b) <u>(2R,5S,10aS,10bS)-2-Aethyl-tetrahydro-10b-hydroxy-5-isopropyl-2-methylamino-8H-oxazolo[3,2-a]pyrrolo[2,1-c]pyrazin-3,6(2H,5H)-dion monomethansulfonat</u>

5,3 g (10 m Mol) Cyclohexan-haltige Kristalle des unter a) erhaltenen Produktes werden in 20 ml abs. Dimethylformamid gelöst, bei 0 ° mit 0,97 ml (15 m Mol) Methansulfonsäure und 2 g 10 %-Pd-auf-Kohle-Katalysator vermischt und 4 Stunden bei 0 ° hydriert, wobei die Hydrieratmosphäre durch 10 N Kaliumhydroxid-Lösung und Phosphorpentoxid zwecks Kohlendioxid-Absorption und Trocknung zirkuliert wird. Danach wird durch Filtererde unter Stickstoffatmosphäre filtriert, das Filtrat im Hochvakuum aus einem Bad von 22 ° eingedampft, der grünliche Rückstand bei 0 ° aus Aceton/Toluol (50 : 50) kristallisiert und die Kristalle bei 22 ° im Hochvakuum getrocknet. Smp.: 108 - 110 ° (Zers.); $[\alpha]_D^{20}$ = - 2,6 ° (1,05 % in Chloroform).

Die folgenden Verbindungen der Formel I können analog dem in Beispiel 1 beschriebenen Verfahren hergestellt werden:

<u>Beispiel 2:</u> <u>2'β-Aethyl-5'α-isopropyl-19-methyl-ergopeptin</u>

Smp.: 164 - 167 ° (aus Isopropylalkohol); $[\alpha]_D^{20}$ = + 147 ° [c = 0,5 in Chloroform : Methanol (90 : 10)].

BAD ORIGINAL

Beispiel 3: 19,2'β,5'α-Trimethyl-ergopeptin

Smp.: 156 - 157 ° (aus Aceton/Wasser); $[\alpha]_D^{20}$ = + 165,5° (c = 0,5 in Chloroform).

Beispiel 4: 9,10α-Dihydro-19,2'β,5'α-trimethyl-ergopeptin

Smp.: 149 - 152 ° (aus Methylenchlorid/Aether); $[\alpha]_D^{20}$ = + 19,6 ° (c = 0,75 in Pyridin).

Beispiel 5: 2'β-Aethyl-9,10α-dihydro-5'α-isopropyl-19-methyl-ergopeptin (Verfahren b)

5,62 g (10 m Mol) 2'β-Aethyl-5'α-isopropyl-19-methyl-ergopeptin und 2,81 g 10 %-Palladium-auf-Aluminiumoxid-Katalysator werden in 80 ml Dimethylformamid, 60 ml Methylenchlorid und 60 ml Essigsäure suspendiert und 8 Stunden bei Zimmertemperatur und Normaldruck hydriert. Nach Filtration durch Filtererde, Abdampfen des Methylenchlorids und der Essigsäure im Vakuum wird zwischen Methylenchlorid und 2 N Ammoniak verteilt und der Rückstand der organischen Phasen aus Methanol kristallisiert und umkristallisiert. Smp.: ca. 180 ° (Zers.).

Analog dem in Beispiel 5 beschriebenen Verfahren kann auch die Verbindung des Beispiels 4 hergestellt werden.

Patentansprüche:

1. Ein auf der Amidbrücke zwischen dem Peptidteil und dem Lysergsäurederivatrest durch einen linearen aliphatischen Kohlenwasserstoffrest substituiertes Peptidergotalkaloid und seine Säureadditionssalze.

2. Eine Verbindung der Formel I,

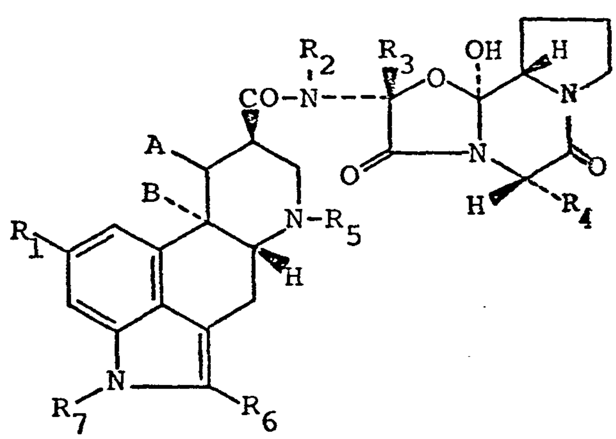

I

worin

$R_1$ für Wasserstoff oder Brom steht,

$R_2$ und $R_3$ unabhängig voneinander eine lineare Alkylgruppe mit 1 bis 4 Kohlenstoffatomen bedeuten,
$R_4$ für Alkyl mit 1 bis 6 Kohlenstoffatomen oder Benzyl steht,
$R_5$ für Alkyl mit 1 bis 4 Kohlenstoffatomen steht,
$R_6$ Wasserstoff oder Halogen mit einer Atomzahl von 9 bis 35 bedeutet,
$R_7$ für Wasserstoff oder Alkyl mit 1 bis 4 Kohlenstoffatomen steht und
A und B zusammen eine einfache Bindung bilden oder
A und B je für Wasserstoff stehen oder
A Wasserstoff und B Methoxy darstellen,

und ihre Säureadditionssalze.

3. Eine Verbindung der Formel I gemäss Anspruch 3, worin
$R_3$ für Aethyl steht,
$R_4$ für Isopropyl steht,
$R_5$ für Methyl steht,
$R_6$ und $R_7$ je Wasserstoff bedeuten und
A und B je Wasserstoff bedeuten oder zusammen eine einfache Bindung bilden.

4. Das 2'β-Aethyl-9,10α-dihydro-5'α-isopropyl-19-methyl-ergopeptin und seine Säureadditionssalze.

5. Verfahren zur Herstellung von auf der Amidbrücke zwischen dem Peptidteil und dem Lysergsäurederivatrest durch einen linearen aliphatischen Kohlenwasserstoffrest substituierten Peptidergotalkaloiden und ihrer Säureadditionssalze, dadurch gekennzeichnet, dass man

a) ein Säureadditionssalz eines entsprechend N-substituierten Aminocyclols mit einem entsprechenden reaktiven Lysergsäurederivat kondensiert oder

b) zur Herstellung der in 9,10-Stellung gesättigten Verbindungen, die frei von unter den Reduktionsbedingungen empfindlichen Substituenten sind, die entsprechenden, in 9,10-Stellung ungesättigten Verbindungen reduziert

und die erhaltenen Verbindungen gewünschtenfalls in ihre Säureadditionssalze überführt.

6. Verfahren zur Herstellung von Verbindungen der Formel I gemäss Anspruch 3 und ihrer Säureadditionssalze, dadurch gekennzeichnet, dass man

a) ein Säureadditionssalz einer Verbindung der Formel II,

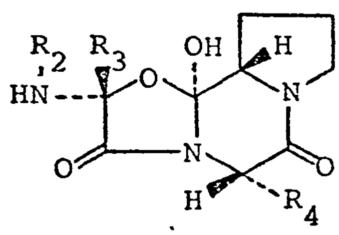

II

worin $R_2$, $R_3$ und $R_4$ wie im Anspruch 3 definiert sind, mit einem reaktiven Säurederivat einer Verbindung der Formel III,

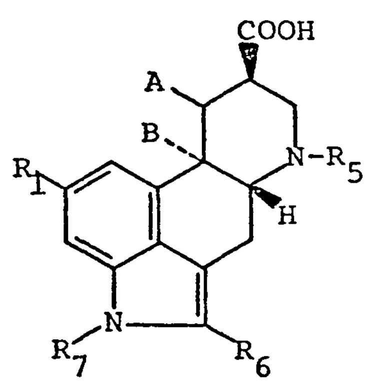

III

worin $R_1$, $R_5$, $R_6$, $R_7$, A und B wie im Anspruch 3 definiert sind, kondensiert oder

b) zur Herstellung von Verbindungen der Formel Ia,

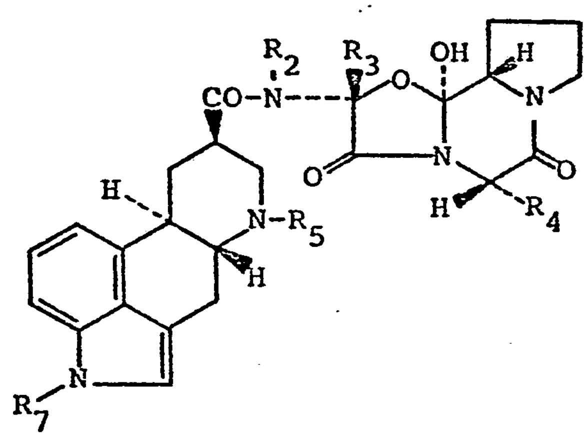

Ia

worin $R_2$, $R_3$, $R_4$, $R_5$ und $R_7$ wie im Anspruch 3 definiert sind, Verbindungen der Formel Ib,

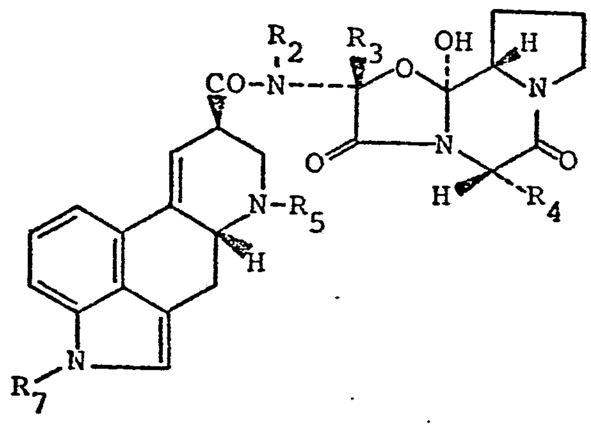

Ib

worin $R_2$, $R_3$, $R_4$, $R_5$ und $R_7$ wie im Anspruch 3 definiert sind, reduziert

und die erhaltenen Verbindungen gewünschtenfalls in ihre Säureadditionssalze überführt.

7. Eine pharmazeutische Zusammensetzung, enthaltend eine Verbindung gemäss einem der Ansprüche 1 bis 4 in Form der freien Base oder eines physiologisch verträglichen Salzes zusammen mit pharmakologisch indifferenten Stoffen.

8. Eine Verbindung gemäss einem der Ansprüche 1 bis 4, zur Anwendung als Pharmazeutikum.

9. Eine Verbindung gemäss einem der Ansprüche 1 bis 4, zur Behandlung der Zerebralinsuffizienz.

10. Ein durch einen linearen aliphatischen Kohlenwasserstoffrest N-substituiertes Aminocyclol eines Peptidergotalkaloids.

Europäisches Patentamt

# EUROPÄISCHER RECHERCHENBERICHT

0026899

Nummer der Anmeldung

EP 80 10 5887

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | CH - A - 610 330 (SANDOZ AG) * Anspruch * -- | |
| A | CH - A - 588 486 (SANDOZ AG) * Anspruch * -- | |
| A | CH - A - 602 766 (SANDOZ AG) * Anspruch * -- | |
| A | CH - A - 602 764 (SANDOZ AG) * Anspruch * -- | |
| A | CH - A - 581 136 (SANDOZ AG) * Anspruch * ---- | |

KLASSIFIKATION DER ANMELDUNG (Int. Cl.3)

A 61 K 31/48
C 07 D 519/02
498/14//
(C 07 D 498/14
263/00
241/00
209/00)

RECHERCHIERTE SACHGEBIETE (Int. Cl.3)

C 07 D 519/02
498/14

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 01-12-1980 | VAN BIJLEN |